Europäisches Patentamt

European Patent Office

Office européen des brevets

(11) Veröffentlichungsnummer: **0 167 784**
**B1**

(12) **EUROPÄISCHE PATENTSCHRIFT**

(45) Veröffentlichungstag der Patentschrift:
19.07.89

(51) Int. Cl.⁴: **C 07 D323/06**, C 08 G   2/08,
C 07 B 63/00

(21) Anmeldenummer: 85106647.2

(22) Anmeldetag: 30.05.85

(54) Verfahren zur Gewinnung von Trioxan aus wässrigen Lösungen durch Hochdruck-Extraktion.

(30) Priorität: 08.06.84 DE 3421300

(43) Veröffentlichungstag der Anmeldung:
15.01.86 Patentblatt 86/03

(45) Bekanntmachung des Hinweises auf die Patenterteilung: 19.07.89 Patentblatt 89/29

(84) Benannte Vertragsstaaten:
AT BE CH DE FR GB IT LI NL SE

(56) Entgegenhaltungen:
EP-A- 0 036 552
GB-A- 1 057 911
US-A- 4 250 331
Zum Stand der Extraktionen mit komprimierten Gasen
Chem. Ing. Techn. 53 (1981) Nr. 7, S.529-542 Verlag
Chemie GmbH D-6940 Weinheim
KIRK-OTHMER, ENCYCLOPEDIA OF CHEMICAL
TECHNOLOGY, THIRD EDITION, SUPPLEMENT
VOLUME, S. 872-893, JOHN WILEY & SONS, 1984
ULLMANNS ECYCLOPÄDIE DER TECHNISCHEN
CHEMIE, 4. Auflage, Band 11, 1976, Weinheim
VERLAG CHEMIE "Erdöl und Erdgas bis Formanzanfarbstoffe", Seiten 699-700
Die Akte enthält technische Angaben, die nach dem
Eingang der Anmeldung eingereicht wurden und die
nicht in dieser Patentschrift enthalten sind.

(73) Patentinhaber: HOECHST AKTIENGESELLSCHAFT
Postfach 80 03 20
D-6230 Frankfurt am Main 80 (DE)

(72) Erfinder: Braun, Gero, Dr.
Händelstrasse 73
D-6100 Darmstadt (DE)
Erfinder: Burg, Karlheinz, Dr.
Eichenweg 18
D-6200 Wiesbaden (DE)
Erfinder: Mück, Karl-Friedrich, Dr.
Wittenberger Strasse 17
D-6200 Wiesbaden (DE)

## Beschreibung

Die Erfindung bezieht sich auf ein Verfahren zur Gewinnung von Trioxan aus verdünnt-wäßrigen, formaldehydhaltigen Lösungen durch Hochdruck-Extraktion. Hierbei können auch sehr verdünnte wäßrige Lösungen mit Extraktionsmitteln im überkritischen oder unterkritischen Zustand behandelt werden und das Trioxan in hochkonzentrierter Form abgeschieden werden.

Nach dem Stand der Technik wird Trioxan für gewöhnlich durch Erhitzen von 30-70 %igen wäßrigen Formaldehydlösungen in Gegenwart von sauren Katalysatoren, z. B. 2 bis 25 %iger Mineralsäure (DE-Patentschrift 1.543.390), oder in Gegenwart von sauren Ionenaustauschern (DE-Patentschrift 1.135.491) hergestellt. Das Trioxan wird dabei destillativ aus dem Reaktionsgemisch entfernt. Dies geschieht entweder in einer dem Reaktor aufgesetzten Kolonne gemäß der US-Patentschrift 2.304.080 oder in einer separaten Kolonne, wie in der GB-Patentschrift 1.012.372 beschrieben. Das trioxanreiche Destillat wird dann z. B. mit Methylenchlorid oder Benzol extrahiert, neutralisiert und anschließend destillative gereinigt, wie z. B. in der DE-Auslegeschrift 1.543.815 beschrieben.

Die Hochdruck-Extraktion von organischen Stoffen u. a. aus wäßrigen Lösungen mittels überkritischer Medien ist zwar schon bekannt. So beschreibt beispielsweise die US-Patentschrift 4.250.331 diese Verfahrensweise bei der Extraktion von organischen Säuren aus verdünnten wäßrigen Lösungen der entsprechenden Salze. Als Extraktionstemperaturen werden 35-200 °C genannt. Trotz teilweise hohen Extraktionstemperaturen und der dabei großen Mengen mitgeschleppten Wassers wird die Abscheidung der gelösten Stoffe nur einstufig durch Druckerniedrigung durchgeführt. Eine Abscheidung eines möglichst wasserfreien Extraktes wird nicht angestrebt. Es werden außerdem nur Extraktionsgrade von 1-23 % erreicht.

Auch in weiteren Veröffentlichungen wird die Hochdruck-Extraktion von organischen Stoffen beschrieben (GB-A-1 057 911, Chem. Ing. Techn. 53 (1981) Nr. 7, Seiten 529-542 und Kirk-Othmer, « Encyclopedia of Chemical Technology » 3. Auflage, Ergänzungsband Seite 872-893 (1984)). Diese Veröffentlichungen befassen sich mit der physikalischen und präparativen Seite des Verfahrens ; weder ein Hinweis auf die erfindungsgemäße Gewinnung von Trioxan aus verdünnten, wäßrigen Lösungen, noch die nebeneinander verlaufende Reaktion zu Trioxan und dessen Extraktion konnten den Entgegenhaltungen entnommen werden.

Den bekannten Verfahren zur Abtrennung von Trioxan aus der Reaktionsmischung oder aus dem Reaktordestillat ist gemeinsam, daß sie umständlich und energieintensiv sind. Weiterhin werden große Mengen von Extraktionsmitteln benötigt, die gesundheitlich bedenklich und umweltbelastend sein können.

Es bestand daher die Aufgabe, gegenüber dem nächstliegenden Stand der Technik ein wirkungsvolles, weniger energieaufwendiges und weniger umweltbelastende Stofftrennverfahren bereitzustellen, mit dem sich eine Abtrennung des entstandenen Trioxans aus der wäßrigen Reaktionslösung nach Beendigung bzw. während der Reaktion erreichen läßt. Die bekannten Verfahren sollten wirtschaftlicher gestaltet werden, indem der Material- und Energiebedarf reduziert wird, der u. a. mit der Verwendung großer Mengen an Extraktionsmitteln verbunden ist.

Die Erfindung betrifft ein Verfahren zur Isolierung von Trioxan durch Behandlung einer verdünnten wäßrigen, formaldehydhaltigen Lösung mit einem Extraktionsmittel, das dadurch gekennzeichnet ist, daß die Lösung, die erhalten wird

(1) nach Beendigung der Reaktion von Formaldehyd zu Trioxan oder

(2) während dieser Reaktion folgenden Verfahrensschritten unterworfen wird :

(a) Behandlung und dadurch Extraktion der wäßrigen Trioxanlösung mit einem Extraktionsmittel in Mengen von 1-10 kg/kg Trioxanlösung, wobei sich das Extraktionsmittel

$(a_1)$ während der Behandlung im überkritischen Zustand in einem Temperaturbereich zwischen der kritischen Temperatur und 200 °C und in einem Druckbereich zwischen dem kritischen Druck und 1000 bar befindet und dabei eine überkritische Lösung bildet oder

$(a_2)$ während der Behandlung im unterkritischen Zustand in Form eines Flüssiggases im Temperaturbereich zwischen der kritischen Temperatur und 0 °C und im Druckbereich zwischen 5 und 500 bar oberhalb des Dampfdruckes des Flüssiggases befindet und eine unterkritische Lösung bildet, und die Lösungen $(a_1)$ und $(a_2)$ Trioxan und Extraktionsmittel sowie mitgeschlepptes Wasser und Formaldehyd enthalten,

(b) Trennung der in dem Extraktionsmittel gelösten Substanzen von dem Extraktionsmittel in einer oder mehreren Stufen durch

$(b_1)$ Reduktion des Druckes und/oder der Temperatur 5/4-5

$(b_2)$ Erhöhung der Temperatur derart, daß Druck und Temperatur stets noch über den jeweiligen kritischen Westen liegen oder

$(b_3)$ Verminderung der Temperatur und/oder des Drucks in den unterkritischen Bereich, wobei die Lösungen in das Extraktionsmittel und in eine Mischung von konzentriertem Trioxan, Formaldehyd und Wasser getrennt werden, die getrennt aus dem Abscheider während des Trennungsschrittes abgezogen werden.

Eine Variante des Verfahrens beinhaltet eine Abtrennung eines großen Teils des mitgeschleppten Wassers und des Formaldehyds durch einen Zwischenabscheider nach der Extraktion der wäßrigen Trioxanlösung und ein Auffangen des Extraktionsmittels, das nach Komprimierung wieder in die Behandlungsstufe A zurückgeführt wird und damit einen kontinuierlichen Prozeß ermöglicht.

Nach einer der beiden erfindungsgemäßen Ausführungsformen soll sich das Extraktionsmittel im überkritischen Zustand befinden. Hierzu liegen die Temperatur und der Druck, unter denen diese erfindungsgemäße Verfahrensvariante durchgeführt wird, mindestens bei der kritischen Temperatur bzw. beim kritischen Druck des jeweiligen Extraktionsmittels, vorzugsweise jedoch darüber. Die Temperaturen liegen zwischen der kritischen Temperatur und 200 °C, vorzugsweise 120 °C. Besonders bevorzugt liegen die Temperaturen zwischen 5 °C und 70 °C oberhalb der kritischen Temperatur. Die entsprechenden Drücke liegen zwischen dem kritischen Druck und 1000 bar, vorzugsweise 400 bar und insbesondere zwischen 30 bar und 300 bar oberhalb des kritischen Druckes. Vorteilhaft ist es dabei, mit einem entsprechenden Extraktionsmittel bei einem solchen Extraktionsdruck zu arbeiten, bei dem die Löslichkeit des zu extrahierenden Trioxans im überkritischen Gas möglichst wenig von der Temperatur abhängt. Auf diese Weise wird vermieden, daß das Trioxan bei der erfindungsgemäß bevorzugt durchgeführten Zwischenabscheidung in größeren Mengen mit abgeschieden wird.

Als Extraktionsmittel können erfindungsgemäß die für die Hochdruck-Extraktion bekannten Verbindungen — ggf. als Gemische — eingesetzt werden, sofern sie gegenüber Trioxan inert sind. Beispielsweise seien hier genannt: aliphatische Kohlenwasserstoffe mit beispielsweise 1 bis 6 Kohlenstoffatomen und halogenhaltige Kohlenwasserstoffe, wie Trifluormethan, Chlordifluormethan, Chlortrifluormethan, Dichlordifluormethan oder Bromtrifluormethan. Auch anorganische Gase wie Kohlendioxid, Schwefeldioxid, Ammoniak, Schwefelhexafluorid oder Distickstoffoxid können hierzu Verwendung finden. Gegebenenfalls können auch als sogenannte Schleppmittel dienende Medien wie Methanol, Ethanol, Dimethylformamid, Aceton, Acetonitril, Benzol, Toluol und dgl. mitverwendet werden.

Bevorzugte Extraktionsmittel sind relativ unpolare Gase, wie Kohlendioxid.

Nach der anderen, weniger bevorzugten erfindungsgemäßen Ausführungsform befindet sich das Extraktionsmittel im unterkritischen Zustand und liegt unter den Extraktionsbedingungen als Flüssiggas vor. Die temperatur liegt in diesem Fall zwischen 0 °C und der kritischen Temperatur, vorzugsweise im Bereich von 0 °C und 5 °C unterhalb der kritischen Temperatur. Die entsprechenden Drücke bewegen sich zwischen 5 bar und 500 bar, vorzugsweise zwischen 10 bar und 150 bar oberhalb des jeweiligen Dampfdruckes des Flüssiggases.

Bei der erfindungsgemäß zu extrahierenden wäßrigen Trioxanlösung handelt es sich um die Reaktionslösung, wie sie nach Beendigung der Reaktion von Formaldehyd zu Trioxan anfällt. Es ist aber auch erfindungsgemäß möglich, die während der Reaktion entstehende Lösung direkt mit dem Extraktionsmittel zu behandeln. Die Trioxankonzentration dieser Trioxanlösung kann in weiten Grenzen schwanken. Üblicherweise liegt die Konzentration im Bereich bis zu 10 Gew.-%, vorzugsweise bei 3 bis 7 Gew.-%, jeweils bezogen auf die gesamte Lösung. Daneben enthalten derartige Lösungen noch erhebliche Mengen an Formaldehyd sowie den sauren Katalysator (Mineralsäure, saurer Ionenaustauscher udgl.). Als Nebenprodukte befinden sich darin im Regelfall auch noch Methanol, Ameisensäure, Methylformiat, Methylal, Dioxymethylendimethylether, Dioxan, Trioxepan, Dimethylbicyclotetroxan, Methylbicyclotetroxan, Bicyclotetroxan, Tetroxan etc.

Die Durchführung des erfindungsgemäßen Verfahrens erfolgt in an sich bekannter Weise, wobei das Extraktionsmittel — sei es im überkritischen Zustand oder als Flüssiggas — vorzugsweise im Kreislauf geführt wird. Dabei ist sowohl eine diskontinuierliche als auch eine kontinuierliche Verfahrensweise möglich. Letzteres kann beispielsweise durch Extraktion der Trioxanlösung in einer Hochdruck-Extraktionskolonne (z. B. Siebbodenkolonne) im Gegenstrom geschehen.

Die Menge des Extraktionsmittels ist nicht kritisch und hängt hauptsächlich von der Trioxankonzentration und der Extraktionszeit ab. Sie liegt bei 1 bis 10 kg Extraktionsmittel/kg Trioxanlösung, vorzugsweise bei 2 bis 4 kg/kg Trioxanlösung.

Auch die Zeit, in der das Extraktionsmittel mit der wäßrigen Trioxanlösung in Kontakt ist, kann in weiten Grenzen schwanken, beispielsweise zwischen 1 und 300 Minuten, vorzugsweise zwischen 5 und 60 Minuten. Die Abscheidung der im Extraktionsmittel gelösten Stoffe kann ein- oder mehrstufig durchgeführt werden und erfolgt durch Absenkung des Druckes und/oder durch Absenkung oder Erhöhung der Temperatur derart, daß Druck und Temperatur noch oberhalb des jeweiligen kritischen Wertes liegen, das Extraktionsmittel sich also weiterhin im überkritischen Zustand befindet. Daneben ist es auch möglich, die Temperatur und den Druck in den unterkritischen Bereich abzusenken und damit auch das Extraktionsmittel in den unterkritischen Bereich überzuführen. Vorzugsweise wählt man dabei die Bedingungen so, daß das Extraktionsmittel noch gasförmig bleibt. Schließlich kann bei der Abscheidung entweder nur die Temperatur oder nur der Druck unter den jeweiligen kritischen Wert erniedrigt werden. Hierbei bleibt das Extraktionsmittel noch gasförmig.

Da im Verlauf der Extraktion oft die Beladung des Extraktionsmittels abnimmt, die Lösefähigkeit des Extraktionsmittels also nich mehr vollständig ausgenutzt wird, kann anch Unterschreiten der maximalen Beladung um z. B. 10 % das teilweise beladene Extraktionsmittel auch zusätzlich durch einen zweiten mit frischer Trioxanlösung gefüllten Extraktionsbehälter geleitet werden.

Besonders im Falle der Hochdruck-Extraktion von sehr verdünnten wäßrigen Trioxanlösungen bei höheren Temperaturen und bei Verwendung von überkritischen Gasen als Extraktionsmittel hat es sich als vorteilhaft erwiesen, eine Zwischenabscheidung vorzunehmen, um hier einen Großteil des mitgeschleppten Wassers und Formaldehyds abzutrennen. Die Zwischenabscheidung kann dabei in der Weise erfolgen, wie vorstehend bei der einstufigen Abscheidung beschrieben. Diese Zwischenabscheidung wird bei einer Temperatur durchgeführt, die zwischen der Extraktionstemperatur und der kritischen Temperatur des betreffenden Fluids, vorzugsweise bei 30 °C bis 80 °C liegt, wobei der Druck dem Extraktionsdruck entspricht. Vorzugsweise wird jedoch auch der Druck in dem Zwischenabscheider unter den Extraktionsdruck abgesenkt, das Extraktionsmittel bleibt dabei jedoch vorzugsweise in gasförmigem Zustand. In dem Zwischenabscheider werden ein Großteil des mitgeschleppten Wassers, zumeist mehr als 90 Gew.-%, wie auch bis zu 90 Gew.-% des mitgeschleppten Formaldehyds abgetrennt.

Durch weitere Temperatur- und/oder Druckabsenkung im Endabscheider, wie oben beschrieben, wird dann ein konzentriertes Trioxan erhalten, das in den meisten Fällen als Kristallbrei anfällt.

Die Zwischenabscheidung kann grundsätzlich auch bei Verwendung von Flüssiggasen als Extraktionsmittel benutzt werden, jedoch ist diese Variante hier nicht bevorzugt. Vielmehr wird in diesem Fall zumeist das Verfahren einstufig durchgeführt, d. h. das beladene Extraktionsmittel wird direkt in einen Abscheider geleitet, da Flüssiggase zumindest bei niedrigen Temperaturen meist nur relativ wenig Wasser lösen. In dem Abscheider wird das Flüssiggas dann verdampft, und die gelösten organischen Stoffe sammeln sich an. Das wiederverflüssigte Gas wird zur weiteren Extraktion erneut eingesetzt. Überraschenderweise ist es dabei möglich, auch bei Vorliegen von recht verdünnten wäßrigen Trioxanlösungen hochkonzentrierte Extrakte zu erhalten. Dies gilt insbesondere dann, wenn die Extraktionstemperatur nicht wesentlich über Raumtemperatur liegt.

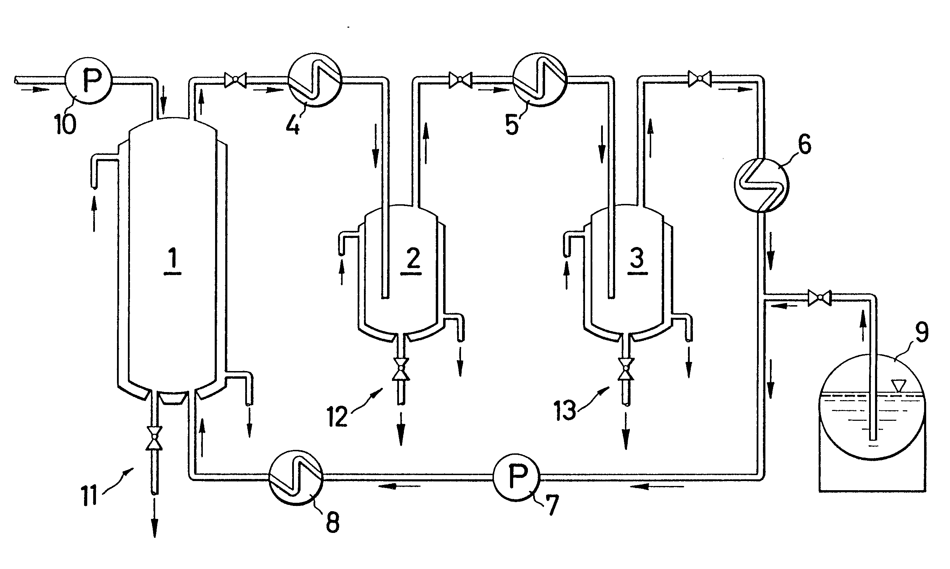

Eine bevorzugte Ausführungsform des erfindungsgemäßen Verfahrens in der Variante mit einem überkritischen Gas wird nachfolgend anhand der beigefügten Figur näher beschrieben :

Der Extraktionsbehälter (1), der vorzugsweise als Siebbodenkolonne oder mehrstufige Blasensäule ausgebildet ist, sowie der Zwischen- (2) und Endabscheider (3) werden aus dem Tank (9) mit dem Extraktionsmittel gefüllt. Dieses wird in dem auf Extraktionstemperatur thermostatisierten Behälter (1) weiter auf den Extraktionsdruck komprimiert. In dem Extraktionsbehälter strömt dem Extraktionsmittel die wäßrige, evtl. Mineralsäure als Katalysator enthaltende Trioxanlösung entgegen, die über die Pumpe (10) eindosiert wird. Dabei belädt sich das überkritische Medium mit dem Trioxan und Wasser im Sinne einer « überkritischen Lösung ». Bei (11) wird die abgereicherte, mit dem Extraktionsmittel behandelte Trioxanlösung abgezogen. Die « überkritische Lösung »

wird demgegenüber durch den Wärmeaustauscher (4) in den Zwischenabscheider (2) überführt, in dem sich bei demselben oder einem niedrigeren Druck wie im Extraktionsbehälter (1) und bei eines niedrigeren Temperatur, der größte Teil des Wassers und des Formaldehyds abscheidet und über (12) ausgetragen wird. Der Rest des Gasstroms gelangt anschließend durch den Wärmeaustauscher (5) in den Endabscheider (3), in dem durch Absenken des Druckes, vorzugsweise auf Werte unterhalb des kritischen Druckes des Gases, das Trioxan mit einer geringen Feuchte abgeschieden wird und über (13), gegebenenfalls nach vorherigem Aufschmelzen, den Endabscheider verläßt. Die Temperatur kann hierbei ebenfalls auf unterkritische Werte gesenkt, aber auch auf gleicher Höhe wie im Zwischenabscheider (2) belassen oder sogar wieder erhöht werden. Der vom Extrakt befreite Gasstrom wird aus dem Behälter (3) durch den Wärmeaustauscher (6) in die Flüssiggaspumpe bzw. den Kompressor (7) geleitet, dort komprimiert und durch den Wärmeaustauscher (8) wieder in den Extraktionsbehälter (1) gefördert. Damit ist der Kreislauf des Extraktionsmittels geschlossen.

Das Wasser, das sich im Zwischenabscheider (2) angesammelt hat, wird über (12) zweckmäßigerweise in ein (in der Figur nicht dargestelltes) Entspannungsgefäß zum Entgasen eingeleitet und das Gas so weit wie möglich durch Abpumpen und Wiederverflüssigen zurückgewonnen. Ein gleiches Vorgehen empfiehlt sich beim Öffnen des Abscheiders (3). Einen Teil des Gases kann man dabei auch dazu verwenden, einen zum Behälter (3) parallelgeschalteten zweiten Abscheider (in der Figur gleichfalls nicht wiedergegeben) teilweise mit Gas zu füllen.

Beispiel 1

In einer Apparatur in Anlehnung an die Fig. mit einem Extraktionsbehälter (2) (ohne Tauchrohr) und einem Abscheider (3) wurden 557 g einer Lösung aus 20,6 % Trioxan, 21,7 % Formaldehyd und 57,7 % Wasser bei einem Druck von 150 bar und einer Temperatur von 80 °C semi-kontinuierlich 5 h mit 2,3 kg/h Trifluormethan behandelt. Von den insgesamt extrahierten 214 g wurden bei 40 bar und 26 °C 190 g mit einem Trioxan-Gehalt von 46,6 % und einem Formaldehyd-Gehalt von 13,2 % in Behälter (3) abgeschieden. Das entspricht einem Anteil von 77 % der gesamten Trioxanmenge der eingesetzten Lösung. Unter Einbeziehung der Verluste durch das Abblasen des Trifluormethans bei Annahme einer vollständigen Abscheidung in (3) würde man einen Extrakt mit einem Trioxan-Gehalt von 50,8 % und einem Formaldehyd-Gehalt von 11,7 % erhalten, entsprechend einem Trioxan-Extraktionsgrad (bezogen auf die Ausgangslösung) von 94,9 %.

Beispiel 2

In einer Apparatur gemäß Beispiel 1 wurden 555 g einer Lösung aus 4,2 % Trioxan, 30 % Formaldehyd und 65,8 % Wasser bein einem Druck von 150 bar und einer Temperatur von 80 °C 6 h mit 1,9 kg/h $CO_2$ behandelt. Von den insgesamt extrahierten 31 g wurden bei 50 bar und 24 °C 21,7 g mit einem Trioxan-Gehalt von 41,9 % und einem Formaldehyd-Gehalt von 13,3 % abgeschieden. Das entspricht einem Anteil von 39,3 % der gesamten Trioxanmenge der eingesetzten Lösung. Unter Einbezieheung der Abblaseverluste bei Annahme einer vollständigen Abscheidung würde man einen Extrakt mit einem Trioxan-Gehalt von 53,7 % und einem Formaldehyd-Gehalt von 30,0 % erhalten, entsprechend einem Trioxan-Extraktionsgrad von 72,1 %.

Beispiel 3

In einer Apparatur in Anlehnung an die Fig. mit einem Rührautoklaven als Extraktionsbehälter (1) (Rührdrehzahl 500 min$^{-1}$) sowie einem Zwischenabscheider (2) und einem Endabscheider (3) wurden 542 g einer Lösung aus 5 % Trioxan, 40 % Formaldehyd und 55 % Wasser bei einem Druck von 200 bar und einer Temperatur von 100 °C 2 h mit 2 kg/h $CO_2$ behandelt. Von den insgesamt extrahierten 73 g wurden durch eine Zwischenabscheidung bei 70 bar und 60 °C 42,5 g mit einem Trioxan-Gehalt von 6,2 % und einem Formaldehyd-Gehalt von 33,6 % abgeschieden. Nach einer weiteren Druck- und Temperatursenkung auf 50 bar und 26 °C wurden in Behälter (3) 14,4 g mit einem Trioxan-Gehalt von 80,3 % und einem Formaldehyd-Gehalt von 5,2 % abgeschieden. Unter Einbeziehung der Abblase-Verluste bei Annahme einer vollständigen Abscheidung in (3) würde man einen Extrakt mit einem Trioxan-Gehalt von 67,2 % und einem Formaldehyd-Gehalt von 11,8 % erhalten, entsprechend einem Trioxan-Extraktionsgrad von 75,7 %. Insgesamt wurden 85,3 % des Trioxans aus der Ausgangslösung extrahiert.

Beispiel 4

In eine Apparatur in Anlehnung an Beispiel 3 ohne Zwischenabscheider (2) wurden 3500 g einer Lösung aus 7 % Trioxan, 40 % Formaldehyd und 53 % Wasser mit einem Druck von 250 bar und einer Temperatur von 80-85 °C 7 Stunden lang mit 2 kg/h $CO_2$ behandelt. Von den insgesamt extrahierten 360 g wurden durch eine Abscheidung bei 45 °C und 60 bar 338 g als Extrakt mit einem Trioxan-Gehalt von 56 % und einem Formaldehyd-Gehalt von 16 % erhalten. Unter Einbeziehung der Abblase-Verluste bei Annahme einer vollständigen Abscheidung in (3) würde man einen Extrakt mit einem Trioxan-Gehalt von 60 % und einem Formaldehyd-Gehalt von 15 % erhalten, entsprechend einem Trioxan-Extraktionsgrad von 84 %.

Beispiel 5

In einem Sichtautoklav von 80 ml Inhalt wurden 30 ml einer Lösung aus 5 % Trioxan, 40 % Formaldehyd und 55 % Wasser bei einem Druck von 100 bar und einer Temperatur von 19 °C 2 h unter Schütteln mit 50 ml flüssigem $CO_2$ behandelt. Nach dem Abtrennen der flüssigen $CO_2$-Phase und Verdampfen des $CO_2$ erhielt man eine Ausbeute von 0,9 g mit einem Trioxan-Gehalt von ca. 75 %. Der Extraktionsgrad betrug damit etwa 45 %.

Beispiel 6

In einer Apparatur gemäß Beispiel 1 wurden 600 g einer Lösung aus 52.5 % Formaldehyd, 41,5 % Wasser und 6 % Schwefelsäure als Katalysator bei einem Druck von 200 bar und einer Temperatur von 100 °C 6 h mit 2 kg/h $CO_2$ behandelt. Dabei bildeten sich etwa 20,4 g Trioxan entsprechend einem Umsatz an Formaldehyd von 6,5 %. Von den ingesamt extrahierten 39 g wurden bei 50 bar und 24 °C 10 g mit einem Trioxan-Gehalt von 25,8 % und einem Formaldehyd-Gehalt von 31,1 % abgeschieden. Unter Einbeziehung der Abblase-Verluste bei Annahme einer vollständigen Abscheidung in (3) würde man einen Extrakt mit einem Trioxan-Gehalt von ca. 19,9 % und einem Formaldehyd-Gehalt von ca. 12,2 % erhalten, entsprechend einem Extraktionsgrad des gebildeten Trioxans von etwa 40,8 %.

Patentansprüche

1. Verfahren zur Isolierung von Trioxan durch behandlung einer verdünnten wäßrigen, formaldehydhaltigen Lösung mit einem Extraktionsmittel, dadurch gekennzeichnet, daß die Lösung, die erhalten wird

(1) nach Beendigung der Reaktion von Formaldehyd zu Trioxan oder

(2) während dieser Reaktion
folgenden Verfahrensschritten unterworfen wird :

(a) Behandlung und dadurch Extraktion der wäßrigen Trioxanlösung mit einem Extraktionsmittel in Mengen von 1-10 kg/kg Trioxanlösung, wobei sich das Extraktionsmittel

(a$_1$) während der Behandlung im überkritischen Zustand in einem Temperaturbereich zwischen der kritischen Temperatur and 200 °C und in einem Druckbereich zweischen dem kritischen Druck und 1000 bar befindet und dabei eine überkritische Lösung bildet oder

(a$_2$) während der Behandlung im unterkritischen Zustand in Form eines Flüssiggases im Temperaturbereich zwischen der kritischen Temperatur und 0 °C und im Druckbereich zwischen 5 und 500 bar oberhald des Dampfdruckes des Flüssiggases befindet und eine unterkritische Lösung bildet, und die Lösungen (a$_1$) und (a$_2$) Trioxan und Extraktionsmittel sowie mitgeschlepptes Wasser und Formaldehyd enthalten,

(b) Trennung der in dem Extraktionsmittel gelösten Substanzen von dem Extraktionsmittel in einer oder mehreren Stufen durch

(b$_1$) Reduktion des Druckes und/oder der Temperatur

(b₂) Erhöhung der Temperatur derart, daß Druck und Temperatur stets noch über den jeweiligen kritischen Werten liegen oder

(b₃) Verminderung der Temperatur und/oder des Drucks in den unterkritischen Bereich,
wobei die Lösungen in das Extraktionsmittel und in eine Mischung von konzentriertem Trioxan, Formaldehyd und Wasser getrennt werden, die getrennt aus dem Abscheider während des Trennungsschrittes abgezogen werden.

2. Verfahren nach Anspruch 1, dadurch gekennzeichnet, daß in Stufe (b) ein großer Teil des mitgeschleppten Wassers und des Formaldehyds durch eine Zwischenabscheidung abgetrennt wird bei einem Druck, der dem Extraktionsdruck entspricht oder niedriger als dieser ist und bei einer Temperatur, die zwischen der Extraktions- und der kritischen Temperatur des betreffenden Fluids liegt.

3. Verfahren zur Isolierung von Trioxan durch Behandlung einer verdünnten wäßrigen, formaldehydhaltigen Lösung mit einem Extraktionsmittel, dadurch gekennzeichnet, daß die Lösung, die erhalten wird

(1) nach Beendigung der Reaktion von Formaldehyd zu Trioxan oder

(2) während dieser Reaktion
folgenden Verfahrensschritten unterworfen wird :

(A) Behandlung und dadurch Extraktion der wäßrigen Trioxanlösung mit einem Extraktionsmittel in Mengen von 1-10 kg/kg Trioxanlösung, wobei sich das Extraktionsmittel

(A₁) während der Behandlung im überkritischen Zustand in einem Temperaturbereich zwischen der kritischen Temperatur und 200 °C und in einem Druckbereich zwischen dem kritischen Druck und 1000 bar befindet und dabei eine überkritische Lösung bildet oder

(A₂) während der Behandlung im unterkritischen Zustand in Form eines Flüssiggases im Temperaturbereich zwischen der kritischen Temperatur und 0 °C und im Druckbereich zwischen 5 und 500 bar oberhalb des Dampfdruckes des Flüssiggases befindet und eine unterkritische Lösung bildet, und die Lösungen (A₁) und (A₂) Trioxan und Extraktionsmittel sowie mitgeschlepptes Wasser und Formaldehyd enthalten,

(B) Abtrennung eines großen Teils des eingeschleppten Wassers und Formaldehyds durch einen Zwischenabscheider, wobei der Druck dem Druck bei der Extraktion entspricht oder tiefer liegt und die Temperatur zwischen der Extraktionstemperatur und der kritischen Temperatur der Lösung liegt,

(C) Trennung der in dem Extraktionsmittel gelösten Substanzen von dem Extraktionsmittel in einer oder mehreren Stufen durch

(C₁) Reduktion des Druckes und/oder der Temperatur

(C₂) Erhöhung der Temperatur derart, daß Druck und Temperatur stets noch über den jeweiligen kritischen Westen liegen oder

(C₃) Verminderung der Temperatur und/oder des Drucks in den unterkritischen Bereich, wobei die Lösungen in das Extraktionsmittel und in eine Mischung von konzentriertem Trioxan, Formaldehyd und Wasser getrennt werden, die aus dem Abscheider abgezogen werden und

(D) schrittweise Auffangen, Komprimieren und Rückführung des Extraktionsmittels in die Behandlungsstufe (A).

4. Verfahren nach einem oder mehreren der Ansprüche 1 bis 3, dadurch gekennzeichnet, daß die überkritischen Bedingungen den Temperaturbereich zwischen der kritischen Temperatur und 120 °C und den Druckbereich zwischen dem kritischen Druck und 400 bar des Extraktionsmittels umfassen.

5. Verfahren nach Anspruch 4, dadurch gekennzeichnet, daß der temperaturbereich 5 bis 70 °C oberhalb der kritischen Temperatur und der Druckbereich 30 bis 300 bar oberhalb des kritischen Druckes liegen.

6. Verfahren nach einem oder mehreren der Ansprüche 1 bis 3, dadurch gekennzeichnet, daß die unterkritischen Bedingungen den Temperaturbereich zwischen 0 und 5 °C unterhalb der kritischen Temperatur des Flüssiggases und den Druckbereich zwischen 10 und 150 bar oberhalb des jeweiligen Dampfdrucks des Flüssiggases umfassen.

7. Verfahren nach einem oder mehreren der Ansprüche 1 bis 6, dadurch gekennzeichnet, daß die Menge an Extraktionsmittel 2-4 kg/kg Trioxanlösung beträgt.

8. Verfahren nach einem oder mehreren der Ansprüche 1 bis 7, dadurch gekennzeichnet, daß die Lösung, die der Extraktion unterworfen wird, eine Trioxankonzentration bis zu 10 Gew.-%, vorzugsweise 3 bis 7 Gew.-%, bezogen auf die gesamte Lösung, aufweist.

9. Verfahren nach einem oder mehreren der Ansprüche 1 bis 8, dadurch gekennzeichnet, daß das Trioxan in konzentrierter Form als Kristallbrei anfällt.

**Claims**

1. A process for the isolation of trioxane by treatment of a dilute aqueous formaldehyde-containing solution with an extracting agent, which comprises the solution which is obtained being subjected

(1) after completion of the reaction of the formaldehyde to give trioxane, or

(2) during this reaction,
to the following process steps :

(a) treatment and, by this means, extraction of the aqueous trioxane solution with an extracting agent in amounts of 1-10 kg/kg of trioxane solution, where the extracting agent

(a₁) is during the treatment in the supercritical state in a temperature range between the critical temperature and 200 °C and in a pressure range between the critical pressure and 1000 bar, and thus forms a supercritical solution, or

(a₂) is during the treatment in the subcritical state in the form of a liquid gas in the temperature range between the critical temperature and 0 °C and in the pressure range between 5 and 500 bar above the vapor pressure of the liquid gas, and forms a subcritical solution, and the solutions (a₁) and (a₂) contain trioxane and extracting agent together with water and formaldehyde which have been carried over,

(b) separation from the extracting agent of the substances which are dissolved in the extracting agent in one or more stages by

(b₁) reducing the pressure and/or the temperature

(b₂) raising the temperature in such a manner that the pressure and temperature are always still above the relevant critical values, or

(b₃) lowering the temperature and/or the pressure into the subcritical range,
where the solutions are separated into the extracting agent and into a mixture of concentrated trioxane, formaldehyde and water, which are drawn off separately from the separator during the separation step.

2. The process as claimed in Claim 1, wherein in stage (b) a large part of the water which has been carried over and of the formaldehyde is separated out by an intermediate separation under a pressure which corresponds to the extraction pressure or is lower than the latter, and at a temperature which is between the extraction temperature and the critical temperature of the relevant fluid.

3. A process for the isolation of trioxane by treatment of a dilute aqueous formaldehyde-containing solution with an extracting agent, which comprises the solution which is obtained being subjected

(1) after completion of the reaction of the formaldehyde to give trioxane, or

(2) during this reaction,
to the following process steps :

(A) treatment and, by this means, extraction of the aqueous trioxane solution with an extracting agent in amounts of 1-10 kg/kg of trioxane solution, where the extracting agent

(A₁) is during the treatment in the supercritical state in a temperature range between the critical temperature and 200 °C and in a pressure range between the critical pressure and 1000 bar, and thus forms a supercritical fluid, or

(A₂) is during the treatment in the subcritical state in the form of a liquid gas in the temperature range between the critical temperature and 0 °C and in the pressure range between 5 and 500 bar above the vapor pressure of the liquid gas, and forms a subcritical solution, and the solutions (A₁) and (A₂) contain trioxane and extracting agent together with water and formaldehyde which have been carried over,

(B) separation out of a large part of the water and formaldehyde which have been carried over by an intermediate separator, where the pressure corresponds to the pressure in the extraction or is lower, and the temperature is between the extraction temperature and the critical temperature of the fluid,

(C) separation from the extracting agent of the substances which are dissolved in the extracting agent in one or more stages by

(C₁) reducing the pressure and/or the temperature

(C₂) raising the temperature in such a manner that the pressure and temperature are always still above the relevant critical values, or

(C₃) lowering the temperature and/or the pressure into the subcritical range,
where the solutions are separated into the extracting agent and into a mixture of concentrated trioxane, formaldehyde and water, which are drawn off from the separator, and

(D) stepwise collection, compression and returning of the extracting agent to treatment stage (A).

4. The process as claimed in one or more of Claims 1 to 3, wherein the supercritical conditions comprise the temperature range between the critical temperature and 120 °C and the pressure range between the critical pressure and 400 bar of the extracting agent.

5. The process as claimed in Claim 4, wherein the temperature range is 5 to 70 °C above the critical temperature and the pressure range is 30 to 300 bar above the critical pressure.

6. The process as claimed in one or more of Claims 1 to 3, wherein the subcritical conditions comprise the temperature range between 0 and 5 °C below the critical temperature of the liquid gas and the pressure range between 10 and 150 bar above the particular vapor pressure of the liquid gas.

7. The process as claimed in one or more of Claims 1 to 6, wherein the amount of extracting agent is 2-4 kg/kg of trioxane solution.

8. The process as claimed in one or more of Claims 1 to 7, wherein the solution which is subjected to the extraction has a trioxane concentration up to 10 % by weight, preferably 3 to 7 % by weight, based on the total solution.

9. The process as claimed in one or more of Claims 1 to 8, wherein the trioxane is produced in concentrated form as a slurry of crystals.

**Revendications**

1. Procédé pour isoler le trioxanne, par traitement d'une solution aqueuse diluée, et contenant du formaldéhyde, à l'aide d'un agent d'extraction, caractérisé en ce que la solution obtenue est,

(1) après la fin de la réaction du formaldéhyde sur le trioxanne, ou

(2) pendant cette réaction,
soumise aux étapes de procédé suivantes :

(a) traitement et, de ce fait, extraction de la solution aqueuse de trioxanne à l'aide d'un agent d'extraction en des quantités de 1 à 10 kg/kg de solution de trioxanne, ce à l'occasion de quoi l'agent d'extraction

(a₁) se trouve pendant le traitement à l'état surcritique, dans un intervalle de températures compris entre la température critique et 200 °C, et dans un intervalle de pressions compris entre la pression critique et 1000 bar, et forme donc une solution surcritique, ou bien

(a₂) se trouve pendant le traitement à l'état sous-critique sous la forme d'un gaz liquéfié dans l'intervalle de températures compris entre la température critique et 0 °C et dans l'intervalle de pressions compris entre 5 et 500 bar au-dessus de la pression de vapeur du gaz liquéfié, et forme une solution sous-critique, les solutions (a₁) et (a₂) contenant du trioxanne et l'agent d'extraction, de même que l'eau entraînée et du formaldéhyde,

(b) séparation, d'avec l'agent d'extraction, des substances dissoutes dans l'agent d'extraction, en une ou plusieurs étapes, par

(b₁) réduction de la pression et/ou de la température,

(b₂) élévation de la température de telle sorte que la pression et la température soient encore toujours supérieures respectivement à la pression et à la température critiques, ou bien

(b₃) diminution de la température et/ou de la pression jusqu'à des valeurs sous-critiques, les solutions étant séparées en l'agent d'extraction et en un mélange de trioxanne concentré, de formaldéhyde et d'eau, qui sont retirés du séparateur pendant l'étape de séparation.

2. Procédé selon la revendication 1, caractérisé en ce que, dans l'étape (b), une partie importante de l'eau entraînée et du formaldéhyde est séparée par une opération de séparation intermédiaire, à une pression correspondant à la pression d'extraction ou étant inférieure à cette dernière, et à une température comprise entre la température d'extraction et la température critique du fluide considéré.

3. Procédé pour isoler le trioxanne, par traitement d'une solution aqueuse diluée, et contenant du formaldéhyde, à l'aide d'un agent d'extraction, caractérisé en ce que la solution obtenue est,

(1) après la fin de la réaction du formaldéhyde sur le trioxanne, ou

(2) pendant cette réaction, soumise aux étapes de procédé suivantes :

(A) traitement et, de ce fait, extraction de la solution aqueuse de trioxanne à l'aide d'un agent d'extraction en des quantités de 1 à 10 kg/kg de solution de trioxanne, ce à l'occasion de quoi l'agent d'extraction

(A₁) se trouve pendant le traitement à l'état surcritique, dans un intervalle de températures compris entre la température critique et 200 °C, et dans un intervalle de pressions compris entre la pression critique et 1 000 bar, et forme donc une solution surcritique, ou bien

(A₂) se trouve pendant le traitement à l'état sous-critique sous la forme d'un gaz liquéfié dans l'intervalle de températures compris entre la température critique et 0 °C et dans l'intervalle de pressions compris entre 5 et 500 bar au-dessus de la pression de vapeur du gaz liquéfié, et forme une solution sous-critique, les solutions (A₁) et

(A₂) contenant du trioxanne et l'agent d'extraction, de même que l'eau entraînée et du formaldéhyde,

(B) séparation d'une grande partie de l'eau entraînée et du formaldéhyde, par un séparateur intermédiaire, la pression correspondant à la pression lors de l'extraction ou étant plus faible, et la température étant comprise entre la température d'extraction et la température critique de la solution,

(C) séparation, d'avec l'agent d'extraction, des substances dissoutes dans l'agent d'extraction, en une ou plusieurs étapes, par

(C₁) réduction de la pression et/ou de la température,

(C₂) élévation de la température de telle sorte que la pression et la température soient encore toujours supérieures respectivement à la pression et à la température critiques, ou bien

(C₃) diminution de la température et/ou de la pression jusqu'à des valeurs sous-critiques, les solutions étant séparées en l'agent d'extraction et en un mélange de trioxanne concentré, de formaldéhyde et d'eau, qui sont retirés du séparateur pendant l'étape de séparation, et

(D) reprise pas à pas, compression et renvoi de l'agent d'extraction dans l'étape de traitement (A).

4. Procédé tel que ci-dessus, caractérisé en ce que les conditions surcritiques englobent l'intervalle de températures entre la température critique et 120 °C, et l'intervalle de pressions compris entre la pression critique et 400 bar de l'agent d'extraction.

5. Procédé selon la revendication 4, caractérisé en ce que l'intervalle de températures se trouve de 5 à 70 °C au-dessus de la température critique, et l'intervalle de pressions se trouve à 30 à 300 bar au-dessus de la pression critique.

6. Procédé selon l'une ou plusieurs des revendications 1 à 3, caractérisé en ce que les conditions sous-critiques englobent l'intervalle de températures compris entre 0 et 5 °C en dessous de la température critique du gaz liquéfié, et l'intervalle de pressions compris entre 10 et 150 bar au-dessus de la pression de vapeur correspondante du gaz liquéfié.

7. Procédé selon l'une ou plusieurs des revendications 1 à 6, caractérisé en ce que la quantité d'agent d'extraction est de 2 à 4 kg/kg de solution de trioxanne.

8. Procédé selon l'une ou plusieurs des revendications 1 à 7, caractérisé en ce que la solution soumise à l'extraction présente une concentration de trioxanne allant jusqu'à 10 % en poids, de préférence de 3 à 7 % en poids, par rapport à la solution totale.

9. Procédé selon l'une ou plusieurs des revendications 1 à 8, caractérisé en ce que le trioxanne est obtenu sous la forme concentrée d'une bouillie cristalline.

EP 0 167 784 B1